# EUROPÄISCHE PATENTSCHRIFT

(11) **EP 1 888 219 B1**
(45) Veröffentlichungstag und Bekanntmachung des Hinweises auf die Patenterteilung: **27.04.2011**
(21) Anmeldenummer: 06707372.6
(22) Anmeldetag: 02.03.2006
(51) Int. Cl.: B01F 17/00, A61K 8/02, A61K 8/06, A61K 8/37, A61K 8/39, A61Q 19/00

(54) **EMULGATORZUSAMMENSETZUNG AUF BASIS VON PENTAERYTHRITESTERN UND ALKOXYLIERTEN NICHTIONISCHEN EMULGATOREN**
EMULSIFIER COMPOSITION BASED ON PENTAERYTHRITOL ESTERS AND ALKOXYLATED NONIONIC EMULSIFIERS
COMPOSITION D'EMULSIFIANT A BASE D'ESTERS DE PENTAERYTHRITE ET D'EMULSIFIANTS NON IONIQUES ALCOXYLES

(30) Priorität: 11.03.2005 DE 102005011334
(43) Veröffentlichungstag der Anmeldung: 20.02.2008
(73) Patentinhaber: Cognis IP Management GmbH, 40589 Düsseldorf (DE)
(72) Erfinder: WEICHOLD, Catherine, 52062 Aachen (DE); GONDEK, Helga, 40589 Düsseldorf (DE); ISSBERNER, Ulrich, 19002 Ambler (US); KAWA, Rolf, 40789 Monheim (DE); GOGET, Caroline, F-75018 Paris (FR)
(86) Internationale Anmeldenummer: PCT/EP2006/001897
(87) Internationale Veröffentlichungsnummer: WO 2006/094701

(56) Entgegenhaltungen:
- EP-A- 0 179 416
- WO-A-03/037293
- DE-A1- 10 025 671
- DE-A1- 19 950 017
- US-B1- 6 576 678
- US-B1- 6 623 746

## Beschreibung

### Gebiet der Erfindung

Die Erfindung betrifft spezielle Emulgatorzusammensetzungen gemäß Anspruch 1 auf Basis von Estern des Pentaerythrits oder Oligomeren des Pentaerythrits und alkoxylierten nichtionischen Emulgatoren. Die Erfindung betrifft auch die Verwendung diese Emulgatorzusammensetzung zur Herstellung von Emulsionen und kosmetische Endformulierungen, die mit Hilfe der Emulgatorzusammensetzung erhalten werden.

### Stand der Technik

Zur Herstellung von kosmetischen und/oder pharmazeutischen Formulierungen stehen dem Fachmann eine Reihe von Wachsen zur Verfügung, die sowohl als Konsistenzgeber - insbesondere durch den Aufbau lamellarer Strukturen - als auch als Co-Emulgatoren wirken.

In Formulierungen, welche nach dem PIT-Verfahren (PIT: Phaseninversionstemperatur) hergestellt werden, kommen als Co-Emulgatoren häufig Mono-/Di-/Triglycerid-Gemische zum Einsatz. Hierbei ist eine genaue Abstimmung des Mono-/Di-/Triglycerid-Verhältnisses als auch des Emulgator/CoEmulgator-Verhältnisses notwendig. Beispielsweise wird in der DE 100 25 671 A1 hervorgehoben, dass ein bestimmter Monoglycerid-Anteil nicht unterschritten werden darf, um besonders feinteilige Emulsionen zu erhalten, die auch bei Temperaturbelastung lagerstabil sind und nicht vergelen. Dies bedeutet, dass zur Herstellung von Emulgator-Zusammensetzungen für PIT-Emulsionen auf Basis von Glyceriden enge Grenzen eingehalten werden müssen. Dies ist jedoch produktionstechnisch nicht unproblematisch. Auch bei der Langzeitlagerung kann sich das Verhältnis der Mono-/Di-/Trigylceride zueinander ändern.

Formulierungen, die nach diesem PIT-Verfahren hergestellt werden, zeichnen sich in der Regel durch eine besonders feine Tröpfchenverteilung und gute Stabilität aus. Bei Verwendung von Mono- und Diestern des Glycerins als Co-Emulgatoren können jedoch inter- und intramolekulare Acyl-Wanderungen auftreten. Hierbei isomerisiert z.B. ein 1-Acyl-Glycerid in ein Gemisch aus 1- und 2-Acyl-Glyceriden oder es findet eine Disproportionierung zu Glycerin und den Diglyceriden 1,2-Diacyl- und 1,3-Diacylglycerid und dem entsprechenden Triglycerid statt (vgl. J.D. Brandner, R.L. Birkmeier, J. Am. Oil. Chem. Soc. 1960, 37, 390-396**;** J.D. Brandner, R.L. Birkmeier, J. Am. Oil. Chem. Soc. 1964, 41, 367-370**).** Das Ausmaß dieser Isomerisierungs- und Disproportionierungs-reaktionen ist zeit-und temperaturabhängig und kann zusätzlich auch von dem für die Herstellung der Glyceride eingesetzten Katalysator abhängen. Das Phänomen der Acyl-Wanderung bei Glyceriden erschwert deren gezielte Synthese und beeinflußt zusätzlich die anwendungstechnischen Eigenschaften. Emulgator-Gemische, die Partialglyceride als Co-Emulgatoren enthalten und zur Herstellung von PIT-Emulsionen eingesetzt werden, zeigen chargenabhängig oft sehr stark variierende Eigenschaften, so dass nicht immer eine Reproduzierbarkeit der Endformulierung gewährleistet werden kann.

US 6 623 746 offenbart ein Verfahren zum Konditionieren von Papier-/Stofftüchern mit einer Emulgatorzusammensetzung, die einen alkoxylierten nichtionischen Emulgator und 50-95% Glyceride enthält. Ester des Pentaerythrits können vorhanden sein, wobei nicht explizit Ester mit unterschiedlichen Acylgruppen erwähnt werden.

Aufgabe der vorliegenden Erfindung war es, Emulgatorzusammensetzungen zur Verfügung zu stellen, die in einem breiten Mischungsverhältnis einsetzbar sind, sich insbesondere zur Herstellung von PIT-Emulsionen eignen und die bei Partialglyceriden als Co-Emulgatoren beobachteten Probleme nicht aufweisen. Eine weitere Aufgabe der vorliegenden Erfindung bestand darin, Emulgatorzusammensetzungen zur Verfügung zu stellen, welche sich zur Herstellung von PIT- und Mikroemulsionen eignen, die eine Partikelgröße von kleiner gleich 1000 nm, insbesondere kleiner gleich 500 nm aufweisen.

### Beschreibung der Erfindung

Gegenstand der Erfindung ist eine Emulgatorzusammensetzung enthaltend (a) wenigstens einen Ester des Pentaerythrits, des Dipentaerythrits, des Tripentaerythrits oder eines beliebigen Gemisches dieser Ester, (b) wenigstens einen alkoxylierten nichtionischen Emulgator und (c) weniger als 10 Gew.-% Wasser, dadurch gekennzeichnet, dadurch gekennzeichnet, dass falls die Zusammensetzung Partialglyceride enthält, diese in einer Menge kleiner gleich 10 Gew.-% bezogen auf die Menge an Pentaerythritester (a) vorliegen.

In einer bevorzugten Ausführungsform der Erfindung enthält die Emulgatorzusammensetzung Partialglyceride in einer Menge von kleiner gleich 8 Gew.%, bevorzugt kleiner gleich 5, besonders bevorzugt kleiner gleich 3, insbesondere kleiner gleich 1 Gew.-% Partialglyceride bezogen auf die Menge an Pentaerythritester (a). In einer bevorzugten Ausführungsform der Erfindung enthält die Emulgatorzusammensetzung keine Partialglyceride.

In einer weiteren Ausführung der vorliegenden Erfindung enthält die Emulgatorzusammensetzung 1 bis 10 Gew.%, bevorzugt 1 bis 8, insbesondere 1 bis 5, insbesondere 1 bis 3 Gew.% Partialglyceride bezogen auf bezogen auf die Menge an Pentaerythritester (a).

Die Begriffe Emulgatorzusammensetzung, Emulgator- Compound und Compound werden synonym verwendet.

Vorzugsweise enthält die erfindungsgemäße Emulgatorzusammensetzung weniger als 5 Gew.-% Wasser, insbesondere weniger als 1 Gew.-% Wasser, besonders bevorzugt weniger als 0,5 Gew.-% Wasser und insbesondere weniger als 0,1 Gew.-% Wasser. Die Emulgatorzusammensetzungen enthalten üblicherweise nur rohstoffbedingte Restmengen an Wasser. Vorzugsweise besteht die Emulgatorzusammensetzung im Wesentlichen aus den Komponenten (a), (b) und (c), wobei lediglich rohstoffbedingte Verunreinigungen enthalten sind.

Der Einsatz der erfindungsgemäßen Compounds ermöglicht eine chargenunabhängige, reproduzierbare Herstellung von PIT Emulsionen. Im Vergleich zu Emulgator-Compounds wie Emulgade® SE-PF enthält das hier beschriebene Compound im Wesentlichen nur zwei Bestandteile. Dies bedeutet "weniger Variable" für die großtechnische Herstellung und somit weniger Unsicherheitsfaktoren durch Rohstoffschwankungen, so dass eine bessere Reproduzierbarkeit bzw. eine leichtere Handhabbarkeit gewährleistet wird. Zusätzlich ist mit der erfindungsgemäßen Emulgatorzusammensetzung in den meisten Fällen keine Optimierung bei der Herstellung der Endformulierungen notwendig, bei der häufig die Konzentration an z.B. alkoxylierten Emulgatoren weiter angepasst werden muß. In vielen Fällen können zudem mit dem erfindungsgemäßen Compound stabilere PIT-Formulierungen - verglichen mit den bisherigen PIT-Formulierungen - erhalten werden.

Im Gegensatz zu den Glyceriden werden insbesondere für die Ester des Pentaerythrits mit identischen Acylresten kaum Disproportionierungen beobachtet und Isomerisierungen führen aufgrund der hohen Symmetrie des Moleküls jeweils zum gleichen Produkt. Die für Glyceride beschriebenen Probleme treten daher bei diesen Estern nicht auf.

Bei der Verwendung bestimmter Ölkomponenten, wie beispielsweise Eutanol® G16 und Eutanol® G (Guerbetalkohole), Cetiol® PGL (enthaltend Guerbetalkohol) und Myritol 331 in PIT Formulierungen wurden Stabilitätsprobleme beobachtet. In den bisherigen PIT-Formulierungen, die diese Ölkomponenten enthalten, konnte daher maximal ein Fünftel der kompletten Ölmenge aus diesen Ölen bestehen. Die Konzentration solcher 'Problemöle' konnte mit der erfindungsgemäßen Emulgatorzusammensetzung um 25 - 50 Gew.-% bezogen auf die Ölphase gesteigert werden.

Der Einsatz der erfindungsgemäßen Emulgatorzusammensetzung bietet auch deutliche sensorische Vorteile, d.h. es können reichhaltigere sprühbare Formulierungen hergestellt werden, welche von der Verteilung auf der Haut, über die Absorption bis zum endgültigen Hautgefühl (Parameter: Klebrigkeit, Glätte, Öligkeit, Wachsigkeit, Weichheit) bessere Ergebnisse zeigen und zu einer höheren Akzeptanz führen.

### Komponente (a)

Erfindungsgemäß bevorzugt sind Emulgatorzusammensetzungen, die dadurch gekennzeichnet sind, dass die Komponente (a) ausgewählt ist aus der Gruppe der C6-C22-Fettsäureester des Pentaerythrits, des Dipentaerythrits, des Tripentaerythrits oder eines beliebigen Gemisches dieser Ester, die einen Schmelzpunkt von wenigstens 30° C aufweisen.

Die Ester können eine einzige Art von Fettsäure-Acylgruppen aufweisen oder ein Gemisch verschiedener Fettsäure-Acylgruppen, die Fettsäuren können verzweigt oder unverzweigt und/oder gesättigt oder ungesättigt sein. Vorzugsweise werden für die Veresterung Fettsäuren/Fettsäuregemische mit einem hohen Gehalt an gesättigten unverzweigten Fettsäuren eingesetzt, insbesondere solche, die aus pflanzlichen Rohstoffquellen stammen. Erfindungsgemäß bevorzugt sind C14-C24-Fettsäuren, insbesondere C14-C20-Fettsäuren. Hierzu zählen z.B. Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure. Eine besonders bevorzugte Ausführungsform enthält als Komponente (a) Ester des Pentaertyhrits, Dipentaerythrits oder Tripentaerythrits, die weniger als 0,3 Gew.-% C17-Fettsäureacylgruppen enthalten.

Eine weitere bevorzugte- Ausführungsform der erfindungsgemäßen Emulgatorzusammensetzung ist dadurch gekennzeichnet, dass die Komponente (a) ein Pentaerythritestergemisch mit einen Anteil von (i) 5 - 35 Gew.-% Monoester, (ii) 20 - 50 Gew.-% Diester und (iii) 25 - 50 Gew.-% Triester, und ggf. Tetraester ist. Besonders bevorzugt ist ein Gehalt an (a) 10 - 25 Gew.-% Monoester, (b) 25 - 40 Gew.- % Diester und (c) 30 - 45 Gew.-% Triester, und ggf. Tetraester und ganz besonders bevorzugt (a) 12-19 Gew.-% Monoester, (b) 25 - 35 Gew.-% Diester, (c) 30- 40 Gew.-% Triester und (d) 6-11 Gew.-% Tetraester.

Eine weitere bevorzugte Ausführungsform der erfindungsgemäßen Emulgatorzusammensetzung ist dadurch gekennzeichnet, dass man die Komponente (a) durch Veresterung mit einem Fettsäuregemisch enthaltend 40 - 50 Gew.-% C16-Fettsäure und 45 - 55 Gew.-% C18-Fettsäure erhält. Die Restmengen des Fettsäuregemisches sind kürzerkettige (≤ C14) und längerkettige (> C18) Fettsäuren. Diese Ester sind bezüglich der sensorischen Eigenschaften überlegen. Erfindungsgemäß bevorzugt geeignet ist ein Ester des Pentaerythrits, der durch Umsetzung von Pentaerythrit mit einem Fettsäuregemisch enthaltend 42 - 48 Gew.-% C16-Fettsäure und 50 - 56 Gew.-% C18-Fettsäure (restliche Mengen: ≤ C14-Fettsäuren und > C18-Fettsäuren) erhalten wird und folgende Esterverteilung aufweist: 12-19 Gew.-% Monoester, (b) 25 - 35 Gew.-% Diester, (c) 30 - 40 Gew.-% Triester und (d) 6-11 Gew.-% Tetraester. Üblicherweise werden pro Mol Pentaerythrit 1.8 - 2.2 Mol des Fettsäuregemisches, vorzugsweise 1.9 - 2.1 Mol, für die Veresterung eingesetzt.

Beispielsweise kann die Herstellung von C16/C18-Fettsäure-Pentaerythritestern erfolgen, indem man pro 1 Mol Pentaerythrit 1.8 - 2.2 Mol, vorzugsweise 1.9 - 2.1 Mol, eines Fettsäuregemisches enthaltend 40 - 50 Gew.-% C16-Fettsäure und 45 - 55 Gew.-% C18-Fettsäure oder ein Rohstoffgemisch mit entsprechender Fettsäureverteilung einsetzt und (a) die Veresterung bei Temperaturen im Bereich von 180°C bis 250°C unter Schutzgasatmosphäre, und ohne Lösungsmittel durchführt, (b) das entstehende Wasser abdestilliert, (c) das erhaltene Reaktionsgemisch im Vakuum solange nachrührt, bis eine Säurezahl von <1 und eine OH-Zahl von 145 - 158 erreicht ist, (d) nicht abreagiertes Pentaerythrit abfiltriert und (e) ggf. eine Nachbehandlung mit Wasserstoffperoxid durchführt. Dem Fachmann sind die Methoden zur Kontrolle und Einstellung der Säurezahl und der OH-Zahl hinlänglich bekannt, so dass an dieser Stelle nicht näher darauf eingegangen werden muss.

Eine weitere Ausführungsform der erfindungsgemäßen Emulgatorzusammensetzung ist dadurch gekennzeichnet, dass die Komponente (a) ein Dipentaerythritestergemisch mit einen Anteil von (i) 5 - 35 Gew.-% Monoester, (ii) 20 - 50 Gew.-% Diester und (iii) 25 - 50 Gew.-% Triester, und ggf. Tetraester ist. Besonders bevorzugt ist ein Gehalt an (a) 10 - 25 Gew.-% Monoester, (b) 25 - 40 Gew.- % Diester und (c) 30 - 45 Gew.-% Triester, und ggf. Tetraester und ganz besonders bevorzugt (a) 12-19 Gew.-% Monoester, (b) 25 - 35 Gew.-% Diester, (c) 30 - 40 Gew.-% Triester und (d) 6-11 Gew.-% Tetraester.

Eine weitere Ausführungsform der erfindungsgemäßen Emulgatorzusammensetzung ist dadurch gekennzeichnet, dass die Komponente (a) ein Tripentaerythritestergemisch mit einen Anteil von (i) 5 - 35 Gew.-% Monoester, (ii) 20 - 50 Gew.-% Diester und (iii) 25 - 50 Gew.-% Triester, und ggf. Tetraester ist. Besonders bevorzugt ist ein Gehalt an (a) 10 - 25 Gew.-% Monoester, (b) 25 - 40 Gew.-% Diester und (c) 30 - 45 Gew.-% Triester, und ggf. Tetraester und ganz besonders bevorzugt (a) 12 -19 Gew.-% Monoester, (b) 25 - 35 Gew.-% Diester, (c) 30 - 40 Gew.-% Triester und (d) 6-11 Gew.-% Tetraester.

Die Erfindung umfasst weiterhin beliebige Gemische der genannten Ester des Pentaeryhrits, Dipentaerythrits und Tripentaerythits.

### Komponente (b)

Prinzipiell können als Komponente (b) nichtionische alkoxylierte Emulgatoren eingesetzt werden, vorzugsweise mit einem HLB-Wert von 10 - 20. Hierzu zählen nichtionische ethoxylierte und propoxylierte Emulgatoren.

Ausführliche Listen der HLB-Werte von Handelsemulgatoren sind dem Fachmann bekannt und finden sich z.B. bei Fiedler, Lexikon der Hilfsstoffe für Pharmazie, Kosmetik und angrenzende Gebiete, Band 9, 1971, Seite 265 - 270; Kirk-Othmer (3. Auflage) Band 8, Seiten 909-918 und Janistyn (3. Auflage) Band 1, Seite 470; und Band 3, Seite 68-78. Die dort aufgelisteten nichtionischen Emulgatoren mit einem HLB-Wert größer 10 sollen Teil der vorliegenden Offenbarung sein. Die Emulgatoren können als Aktivsubstanz oder in Form wässriger Lösungen eingesetzt werden.

Zur Gruppe der besonders geeigneten nicht-ionischen Emulgatoren/Tenside mit einem HLB Wert von wenigstens 10 gehören entsprechende alkoxylierte Verbindungen, die ausgewählt sind aus:
(1) Anlagerungsprodukte von Ethylenoxid und/oder Propylenoxid an lineare und verzweigte Fettalkohole mit 8 bis 40 C-Atomen, an lineare und verzweigte Fettsäuren mit 12 bis 40 C-Atomen und an Alkylphenole mit 8 bis 15 C-Atomen in der Alkylgruppe.
(2) C_{12/18}-Fettsäuremono- und -diester von Anlagerungsprodukten von Ethylenoxid und/oder Propylenoxid an Glycerin.
(3) Ethylenoxid- und/oder Propylenoxidanlagerungsprodukte an Glycerinmono- und -diester und Sorbitanmono- und -diester von gesättigten und ungesättigten Fettsäuren mit 6 bis 22 Kohlenstoffatomen
(4) Ethoxylierte und propoxylierte Alkylmono- und -oligoglycoside mit 8 bis 22 Kohlenstoffatomen im Alkylrest
(5) Anlagerungsprodukte von Ethylenoxid und/oder Propylenoxid an Ricinusöl und/oder gehärtetes Ricinusöl.

Einzelne Beispiele (vgl. Kirk-Othmer) für nichtionische Emulgatoren/Tenside mit einem HLB-Wert von mindestens 10 sind POE (5) Sorbitanmonooleat (POE = Polyoxyethylen; PEG = Polyethylenglycol), POE (40) Sorbitolhexaoleat, PEG 400 Dilaurat, POE (5) Nonylphenol(ether), POE (20) Sorbitantristearat, POP/POE Kondensate, POE (6) Nonylphenol(ether), POE (20) Lanolin (ether und ester), POE (20) Sorbitantrioleat, POE (8) Stearinsäure (Monoester), POE (50) Sorbitolhexaoleat, POE (6) Tridecylalkohol (ether), PEG 400 Monostearat, POE (8) Nonylphenol (ether), POE (10) Stearylalkohol (ether), POE (8) Tridecylalkohol (ether), POE (8) Laurinsäure (monoester), POE (10) Cetylalkohol (ether), acetyliertes POE (10) Lanolin, POE (20) Glycerolmonostearat, PEG 400 Monolaurat, POE (16) Lanolinalkohol (ether), POE (4) Sorbitanmonolaurat, POE (10) Nonylphenol (ether), POE (15) lange Ölfettsäuren (ester), POE (10) Octylphenol (ether), PEG 600 Monostearat, tertiäre Amine: POE Fettamine; POE (24) Cholesterol, POE (14) Nonylphenol (ether), POE (12) Laurylalkohol, POE (20) Sorbitanmonostearat, Sucrosemonolaurat, POE (20) Sorbitan Monooleat, acetyliertes POE (9) Lanolin, POE (20) Stearylalkohol, POE (20) Oleylalkohol (ether), PEG 1000 Monooleat, POE (20) Talgamine, POE (20) Sorbitanmonopalmitat, POE (20) Cetylalkohol (ether), POE (25) Propyleneglycolmonostearat, POE (20) Nonylphenol (ether), PEG (1000) Monolaurat, POE (20) Sorbitanmonolaurat, POE (23) Laurylalkohol (ether), POE (40) Stearinsäure (Monoester), POE (50) Lanolin (ether und ester), POE (25) Sojasterol, POE (30) Nonylphenol (ether), PEG 4000 Distearat, POE (50) Stearinsäure (Monoester), POE (70) Dinonylphenol (ether), POE (20) Rizinusöl (ether, ester), N-cetyl-N-ethyl-Morpholiniumethylsulfat, etc.

Eine bevorzugte Ausführungsform der erfindungsgemäßen Emulgatorzusammensetzung ist dadurch gekennzeichnet, dass das der alkoxylierte nichionische Emulgator (b) ausgewählt ist aus der Gruppe der ethoxylierten nichtionischen Emulgatoren, vorzugsweise aus der Gruppe der C12-C24-Fettalkoholethoxylate oder einem beliebigen Gemisch von C12-C24-Fettalkoholethoxylaten.

Vorzugsweise handelt es sich um C12-C24-Fettsäureethoxylte mit einem HLB-Wert von wenigstens 10, besonders bevorzugt C16-C22 Fettsäureethoxylate. Hierzu gehören beispielsweise Ceteareth-12, Ceteareth-20, Ceteareth-30, die von der Cognis Deutschland GmbH & Co. KG unter der Bezeichnung Eumulgin^{®} B1, Eumulgin^{®}B2 bzw. Eumulgin^{®} B3 im Handel sind, sowie Beheneth-10 (Eumulgin^{®} BA 10) und Beheneth-25 (Eumulgin^{®} BA 25).

In einer weiteren bevorzugten Ausführungsform der erfindungsgemäßen Emulgatorzusammensetzung variiert das Gewichtsverhältnis von (a) : (b) zwischen 20 : 80 - 80 : 20, vorzugsweise 25 : 75 - 75 : 25, vorzugsweise 40 : 60 - 60 : 40, besonders bevorzugt 35 : 65 - 65 : 35. In dieser Zusammensetzung werden besonders stabile und feinteilige Emulsionen erhalten.

### Gewerbliche Anwendbarkeit

Die erfindungsgemäßen Emulgatorzusammensetzungen erlauben die Herstellung besonders feinteiliger und stabiler Emulsionen. Ein weiterer Gegenstand der Anmeldung ist daher die Verwendung der erfindungsgemäßen Emulgatorzusammensetzung in Emulsionen sowie Verwendung als selbstemulgierende Grundlage zur Herstellung von Emulsionen, insbesondere in kosmetischen und/oder pharmazeutischen Zubereitungen. Unter dem Begriff "selbstemutgierend" wird erfindungsgemäß verstanden, dass sich die Zusammensetzung ohne Scherkräfte, durch einfaches mechanisches Rühren in Endformulierungen einarbeiten lässt.

Die erfindungsgemäßen Emulgatorzusammensetzungen eignen sich besonders gut zur Herstellung von PIT- und Mikroemulsionen, vorzugsweise mit einer mittleren Tröpfchengrößenverteilung von kleiner gleich, bevorzugt kleiner 1000 nm, bevorzugt kleiner gleich, bevorzugt kleiner 500 nm, ganz besonders bevorzugt kleiner gleich, bevorzugt kleiner 250 nm, bevorzugt kleiner gleich, bevorzugt kleiner 200 nm.

Die Partikelgröße von Emulsionen kann visuell bestimmt werden durch Vergleich mit der Partikelgröße von Standardemulsionen verglichen. Zur Bestimmung der Partikelgröße einer Standardemulsionen wird mittels Laserbeugung ein Beugungsmuster ermittelt. Aus den Lichtintensitäten dieser Beugungsmuster wird dann mittels der Fraunhofer-Theorie die Teilchengrößenverteilung errechnet (Sympatec Helos). Die Partikelgrössen können mit einem Coulter® LS Gerät bestimmt werden durch Verdünnung mit Wasser und anschliessendem Einspritzen dieser Verdünnung in das Gerät.

Die erfindungsgemäßen Emulgatorzusammensetzungen eigen sich besonders gut zur Herstellung von versprühbaren Zubereitungen.

Ein weiterer Gegenstand der Anmeldung ist ein Verfahren zur Herstellung von Emulsionen, wobei man (a) eine Emulgatorzusammensetzung gemäß einem der Ansprüche 1 bis 6 in einer Ölphase, die ggf. weitere öllösliche Komponenten enthält, dispergiert und diese auf eine Temperatur oberhalb der Phaseninversionstemperatur erwärmt, (b) eine wässrige Phase enthaltend ggf. wasserlösliche Bestandteile ebenfalls auf eine Temperatur oberhalb der Phaseninversionstemperatur erwärmt, und dann (c) die Ölphase und die wässrige Phase zusammen unter Rühren auf Raumtemperatur abkühlt. Hierbei ist es nicht notwendig Scherkräfte anzuwenden, einfaches mechanisches Rühren ist ausreichend.

Gegenstand sind auch kosmetische Zusammensetzungen, die 0,5 bis 40 Gew.-%, bevorzugt 2,0 - 20 Gew.-% einer Emulgatorzusammensetzung gemäß wenigstens einem der Ansprüche 1 bis 6 enthalten. Besonders bevorzugt sind kosmetische Zusammensetzungen, die 0,5 bis 40 Gew.-%, bevorzugt 2,0 bis 20 Gew.-% einer Emulgatorzusammensetzung gemäß wenigstens einem der Ansprüche 2 bis 6 enthalten. Vorzugsweise enthalten die kosmetischen Zusammensetzungen zusätzlich wenigstens eine bei 20°C flüssige Ölkomponente. Besonders bevorzugt sind kosmetische Zusammensetzung, die (a) 0,5 bis 40 Gew.-%, bevorzugt 2,0 - 20 Gew.-% der einer Emulgatorzusammensetzung gemäß wenigstens einem der Ansprüche 1 bis 6, (b) 5-30 Gew.-% wenigstens einer bei 20 °C flüssigen Ölkomponente und (c) Wasser enthalten.

Ein weiterer Gegenstand der vorliegenden Erfindung betrifft kosmetischen Zubereitungen, die 0,5 bis 40 Gew.-%, bevorzugt 2,0 - 20 Gew.-% einer Emulgatorzusammensetzung gemäß wenigstens einem der Ansprüche 1 bis 6 enthalten und mindestens einen UV-Lichtschutzfilter.

Ein weiterer Gegenstand der vorliegenden Erfindung betrifft kosmetischen Zubereitungen, die 0,5 bis 40 Gew.-%, bevorzugt 2,0-20 Gew.-% einer Emulgatorzusammensetzung gemäß wenigstens einem der Ansprüche 1 bis 6 enthalten und mindestens ein Insekten-Repellent.

Ein weiterer Gegenstand der vorliegenden Erfindung betrifft kosmetischen Zubereitungen, die 0,5 bis 40 Gew.-%, bevorzugt 2,0 - 20 Gew.-% einer Emulgatorzusammensetzung gemäß wenigstens einem der Ansprüche 1 bis 6 enthalten und mindestens einen Selbstbräuner.

Ein weiterer Gegenstand der vorliegenden Erfindung betrifft kosmetischen Zubereitungen, die 0,5 bis 40 Gew.-%, bevorzugt 2,0 - 20 Gew.-% einer Emulgatorzusammensetzung gemäß wenigstens einem der Ansprüche 1 bis 6 enthalten und mindestens einen desodorierenden Wirkstoff.

In erfindungsgemäßen kosmetischen Zubereitungen können in allen dem Fachmann bekannten Formen, wie beispielsweise Creme, Lotion etc. vorliegen. In einer bevorzugten Ausführungsform liegen die genannten kosmetischen Zubereitungen in Form einer versprühbaren Formulierung vor.

### Ölkomponenten

Die erfindungsgemäßen Zubereitungen enthalten eine wässrige Phase und eine Ölphase, die weitere Hilfs- und Zusatzstoffe enthalten können. Der Anteil der wässrigen Phase, inklusive wasserlöslicher Wirkstoffe, liegt üblicherweise im Bereich von 20 - 90 Gew.-% bezogen auf die Gesamtzusammensetzung, der Anteil der Ölphase bei 1 - 70 Gew.-% bezogen auf die Gesamtzusammensetzung. Die Ölphase kann sich aus einer Ölkomponente oder einem beliebigen Gemisch von Ölkomponenten zusammensetzen, sowie aus öllöslichen Wirkstoffen.

Als Ölkomponenten sind beispielsweise die nachstehend genannten Verbindungsklassen geeignet: Guerbetalkohole auf Basis von Fettalkoholen mit 6 bis 18, vorzugsweise 8 bis 10 Kohlenstoffatomen, z.B. 2-Ethylhexanol oder 2-Octyldodecanol; Ester von linearen oder verzweigten, gesättigten oder ungesättigten C₆-C₂₄-Fettsäuren mit linearen oder verzweigten, gesättigten oder ungesättigten C₆-C₂₄-Fettalkoholen. Beispielhaft seien genannt Hexyllaurat, Ethylhexylstearate, Myristylisostearat, Myristyloleat, Cetearylisononanoate, Cetylisostearat, Cetyloleat, Stearylisostearat, Stearyloleat, Isostearylmyristat, Isostearylpalmitat, Isostearylstearat, Isostearylisostearat, Isostearyloleat, Oleylmyristat, Oleylisostearat, Oleyloleat, Oleylerucat, Erucylisostearat, Erucyloleat, Cococaprylat/caprat. Weitere geeignete Ester sind z.B. Ester von C₁₈-C₃₈-Alkylhydroxycarbonsäuren mit linearen oder verzweigten, gesättigten oder ungesättigten C₆-C₂₂-Fettalkoholen, Ester von linearen und/oder verzweigten, gesättigten oder ungesättigten Fettsäuren mit mehrwertigen Alkoholen (wie z. B. Propylenglycol, Dimerdiol oder Trimertriol) und/oder Guerbetalkoholen, Triglyceride oder Triglyceridmischungen, flüssige Mono-/Di-/Triglyceridmischungen, Ester von C₆-C₂₂-Fettalkoholen und/oder Guerbetalkoholen mit aromatischen Carbonsäuren, insbesondere Benzoesäure (z. B. Finsolv® TN), Ester von C₂-C₁₂-Dicarbonsäuren mit linearen oder verzweigten, gesättigten oder ungesättigten Alkoholen mit 1 bis 22 Kohlenstoffatomen oder Polyolen mit 2 bis 10 Kohlenstoffatomen und 2 bis 6 Hydroxylgruppen. Geeignet sind auch pflanzliche Öle, Triglyceridmischungen, substituierte Cyclohexane, lineare symmetrische oder unsymmetrische Dialkylcarbonate (z.B. Cetiol® CC), Guerbetcarbonate auf Basis von Fettalkoholen mit 6 bis 18, vorzugsweise 8 bis 10 C-Atomen, lineare oder verzweigte, symmetrische oder unsymmetrische Dialkylether mit 6 bis 22 Kohlenstoffatomen pro Alkylgruppe, wie z. B. Di-n-octyl Ether (Cetiol® OE), Ringöffnungsprodukte von epoxidierten Fettsäureestern mit Polyolen, Kohlenwasserstoffe wie Paraffin- oder Mineralöle, Oligo- oder Polyalphaolefine. Die Dialkylcarbonate und Dialkylether können symmetrisch oder unsymmetrisch, verzweigt oder unverzweigt, gesättigt oder ungesättigt sein und lassen sich nach Reaktionen, die aus dem Stand der Technik hinlänglich bekannt sind, herstellen. Geeignete Siliconverbindungen sind beispielsweise Dimethylpolysiloxane, Methylphenylpolysiloxane, cyclische Silicone (Cyclomethicon) sowie amino-, fettsäure-, alkohol-, polyether-, epoxy-, fluor-, glykosid- und/oder alkylmodifizierte Siliconverbindungen. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

In einer bevorzugten Ausführungsform der Erfindung ist die Ölkomponenten ausgewählt aus der Gruppe bestehend aus Dialkylcarbonaten und Dialkylethern.

Als Dialkylcarbonate besonders geeignet sind Verbindungen der Formel (I)

(I) R₁O(CH₂CH₂O)ₙ-CO-(OCH₂CH₂)ₘ-OR₂

in der R₁ für einen linearen Alkyl- und/oder Alkenylrest mit 6 bis 22 Kohlenstoffatomen, einen 2-Ethylhexyl-, Isotridecyl- oder Isostearylrest oder einen Rest, der sich von einem Polyol mit 2 bis 15 Kohlenstoffatomen und mindestens zwei Hydroxylgruppen ableitet, R₂ für R₁ oder einem Alkylrest mit 1 bis 5 Kohlenstoffatomen steht und n und m unabhängig voneinander 0 oder Zahlen von 1 bis 100 bedeuten.

Als Dialkylether besonders geeignet sind Verbindungen der allgemeinen Formel (II):

(II) R₃-O-R₄

in der R₃ und R₄ unabhängig voneinander für einen linearen oder verzweigten Alkyl- und/oder Alkenylrest mit 6 bis 22, vorzugsweise 6 bis 12, vorzugsweise 16 bis 22, vorzugsweise 8 bis 18 und insbesondere 12 bis 18, Kohlenstoffatomen steht. Die Ether können asymmetrisch, vorzugsweise aber symmetrisch aufgebaut sein. Typische Beispiele sind Di- n-octylether,Di-i-octylether und Di-n-stearylether. In einer bevorzugten Ausführungsform wird als Dialkylether Di- n-octylether (erhältich unter dem Handelsnamen Cetiol®OE) und/oder Di-i-octylether eingesetzt.

Eine bevorzugte Ausführungsform der Erfindung betrifft kosmetische Zubereitungen, die (a) 0,5 - 40, bevorzugt, 2,0 - 20 Gew.-% der einer Emulgatorzusammensetzung gemäß wenigstens einem der Ansprüche 1 bis 6, (b) 5 - 30 Gew.-% wenigstens einer bei 20 °C flüssigen Ölkomponente und (c) Wasser enthalten, wobei die wenigsten eine bei 20 °C flüssigen Ölkomponente ausgewählt ist aus der Gruppe bestehend aus Dialkylcarbonaten und Dialkylethern.

Erfindungsgemäß einsetzbar sind auch lineare Kohlenwasserstoffe mit einer Kettenlänge 8 bis 40 C-Atomen, die verzweigt oder unverzweigt, gesättigt oder ungesättigt sein können. Unter diesen sind verzweigte, gesättigte C8-C40-Alkane bevorzugt. Es können sowohl Reinsubstanzen eingesetzt werden als auch Substanzgemische. Üblicherweise handelt es sich um Substanzgemische verschiedener isomerer Verbindungen. Zusammensetzungen, die Alkane mit 10 bis 30, vorzugsweise 12 bis 20, und besonders bevorzugt 16 bis 20 Kohlenstoffatome aufweisen, sind besonders geeignet, und unter diesen ein Gemisch aus Alkanen, welches wenigstens 10 Gew.-% verzweigte Alkane bezogen auf die Gesamtmenge der Alkane enthält. Vorzugsweise handelt es sich um verzweigte, gesättigte Alkane. Besonders gut geeignet sind Gemische aus Alkanen, welche mehr als 1 Gew.-% 5,8-Diethyldodecan und/oder mehr als 1 Gew.-% Didecen enthalten.

Alle Ölkomponenten können, sofern sie bei 20 °C flüssig sind, als Komponente c) der erfindungsgemäßen kosmetischen Zubereitungen vorliegen. Sofern die Ölkomponenten nicht bei 20 °C flüssig sind, können sie als (weiterer) Ölkörper in den kosmetischen Zubereitungen vorliegen.

### Weitere fakultative Hilfs- und Zusatzstoffe

Je nach Applikationszweck können die kosmetischen Formulierungen eine Reihe weiterer Hilfs- und Zusatzstoffe enthalten, wie beispielsweise Verdickungsmittel, Überfettungsmittel, Stabilisatoren, Polymere, Lecithine, Phospholipide, biogene Wirkstoffe, UV-Lichtschutzfaktoren, Antioxidantien, Deodorantien, Filmbildner, Quellmittel, Insektenrepellentien, Hydrotrope, Solubilisatoren, Konservierungsmittel, Parfümöle, Farbstoffe etc., die nachstehend exemplarisch aufgelistet sind. Die Mengen der jeweiligen Zusätze richten sich nach der beabsichtigten Verwendung.

Als **Verdickungsmittel** eignen sich beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyvinylalkohol, Polyvinylpyrrolidon und Bentonite wie z. B. Bentone^{®} Gel VS-5PC (Rheox).

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z. B. Wärme wieder abzugeben. UV-B-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher wie in der EP 0693471 B1 beschrieben;
➢ 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester;
➢ Ester der Zimtsäure, vorzugsweise 4-Methoxyzimtsäure-2-ethylhexylester, 4-Methoxy-zimtsäurepropylester, 4-Methoxyzimtsäureisoamylester, 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-iso-propylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexyl-ester;
➢ Triazinderivate, wie z.B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ Sulfonsäurederivate von Benzophenonen, vorzugsweise 2-Hydroxy-4-methoxybenzo-phenon-5-sulfonsäure und ihre Salze;
➢ Sulfonsäurederivate des 3-Benzylidencamphers, wie z.B. 4-(2-Oxo-3-bomylidenmethyl)-benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzoyl]-, Hexyl Ester (Uvinul® A plus). Die UV-A und UV-B-Filter können selbstverständlich auch in Mischungen eingesetzt werden. Besonders günstige Kombinationen bestehen aus den Derivaten des Benzoylmethans,, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Besonders geeignete Breitspektrum-Sonnenfilter sind 2,2'-Methylenebis [4-(1,1,3,3-Tetramethylbutyl)-6-(2H-Benzotriazol-2-yl)Phenol] (Tinosorb M) und Phenol, 2,2'-[6-(4-Methoxyphenyl)-1,3,5-Triazine-2,4-Diyl]Bis[5-[(2-Ethylhexyl)Oxy]- (Tinosorb M).

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 543 (1996**) sowie** Parf.Kosm. 3, 11 (1999**)** zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Unter biogenen **Wirkstoffen** sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

**Desodorierende Wirkstoffe** wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker. Als Antitranspirant-Wirkstoffe kommen z. B. Aluminiumchlorhydrate, Aluminium-Zirkonium-Chlorohydrate sowie Zinksalze in Frage. Diese wirken wahrscheinlich über den partiellen Verschluß der Schweißdrüsen durch Eiweiß- und/oder Polysaccharidfällung. Neben den Chlorhydraten können auch Aluminiumhydroxylactate sowie saure Aluminium/Zirkoniumsalze eingesetzt werden. Unter der Marke Locron^{®} der Clariant GmbH, befindet beispielsweise sich ein Aluminiumchlorhydrat im Handel, das der Formel [Al₂(OH)₅Cl]·2,5 H₂O entspricht, und dessen Einsatz besonders bevorzugt ist. Ebenso erfindungsgemäß bevorzugt ist der Einsatz von Aluminium-Zirkonium-Tetrachlorohydrex-Glycin-Komplexen, die beispielsweise von Reheis unter der Bezeichnung Rezal^{®} 36G vermarktet werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen^{®} C.A.T., Cognis Deutschland GmbH). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und β-Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester. Antibakterielle Wirkstoffe, welche die Keimflora beeinflussen und schweißzersetzende Bakterien abtöten bzw. in ihrem Wachstum hemmen, können ebenfalls in den Zusammensetzungen enthalten sein. Beispiele hierfür sind Chitosan, Phenoxyethanol und Chlorhexidingluconat. Besonders wirkungsvoll hat sich auch 5-Chlor-2-(2,4-dichlorphenoxy)-phenol erwiesen, das unter der Bezeichnung Irgasan^{®} von der Ciba-Geigy, Basel/CH vertrieben wird.

Als **Insekten-Repellentien** kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage.

Als **Selbstbräuner** eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Als **Konservierungsmittel** eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als **Parfümöle** seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Als **Farbstoffe** können die für kosmetische Zwecke geeigneten und zugelassenen Substanzen verwendet werden. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Diese Farbstoffe werden üblicherweise in Konzentrationen von 0,001 bis 0,1 Gew.-%, bezogen auf die gesamte Mischung, eingesetzt.

Die kosmetischen Zusammensetzungen eignen sich auch zur Beschichtung verschiedenartiger Unterlagen. Ein weiterer Gegenstand der Anmeldung ist die Verwendung der erfindungsgemäßen Zusammensetzungen, insbesondere als Avivagemittel, zur Beschichtung von Vliesen, Geweben, Kosmetik- und Haushaltstüchem sowie Substrate, die mit diesen kosmetischen Zusammensetzungen beschichtet sind.

Beispiele für beschichtete Substrate sind: Wipes zur Körperpflege und Körperhygiene, Abschminktücher, beschichtet Wattepads, Wipes mit Sonnenschutzformulierungen oder Insektenschutzformulierungen, etc.

Die nachfolgenden Beispiele betreffen Untersuchungen zu Emulsionen, die auf Basis der erfindungsgemäßen Emulgatorzusammensetzungen hergestellt wurden, im Vergleich zu Emulsionen, die mit anderen marktüblichen Emulgatoren hergestellt wurden.

### Beispiele

**Tabelle 1**

| Die Rezepturen 1 - 2 in der Tabelle 1 sind erfindungsgemäß, die Rezepturen V1 - V3 dienen zum Vergleich. Die Mengenangaben beziehen sich auf Gew.-% der handelsüblichen Substanzen in der Gesamtzusammensetzung. | | | | | |
|---|---|---|---|---|---|
| **Inhaltstoffe** | **V1** | **V2** | **V3** | **1** | **2** |
| Emulgade® SE-PF | 5 | | | | |
| Eumulgin® B1 | | 1,5 | 1,5 | 2,5 | 3,5 |
| Eumulgin® B2 | | 1,5 | 1,5 | 0,5 | |
| Cutina® MD | | 3,0 | | | |
| Cutina® GMS-V | | | 3,0 | | |
| Cutina® PES | | | | 3,0 | 2,5 |
| Cetiol® OE | 5,0 | 5,0 | 5,0 | 5,0 | 5,0 |
| Cetiol® LC . | 5,0 | 5,0 | 5,0 | 5,0 | 5,0 |
| Glycerin | 3,0 | 3,0 | 3,0 | 3,0 | 3,0 |
| Wasser, Konservierungsmittel | ad 100 | ad 100 | ad 100 | ad 100 | ad 100 |
| | | | | | |
| Phasenstabilität bei -5°C/RT/40°C | | | | | |
| Nach 1 Woche | 1/5/5 | 5/5/5 | 5/5/5 | 1/1/1 | 1/1/1 |
| Nach 2 Wochen | 1/5/5 | 5/5/5 | 5/5/5 | 1/1/1 | 1/1/1 |
| Nach 4 Wochen | 1/5/5 | 5/5/5 | 5/5/5 | 1/1/1 | 1/1/1 |
| Nach 8 Wochen | 1/5/5 | 5/5/5 | 5/5/5 | 1/1/1 | 1/1/1 |
| Nach 12 Wochen | 1/5/5 | 5/5/5 | 5/5/5 | 1/1/1 | 1/1/1 |

| | | | | | |
|---|---|---|---|---|---|
| RT = Raumtemperatur 20°C Legende: Bewertungskriterien für die visuelle Phasenstabilität: 1 - stabil; 2 - geringfügige Trennung; 3 - leichte Trennung; 4 - deutliche Trennung; 5 - Trennung | | | | | |

**Tabelle 2**

| Die Rezepturen 3 - 6 in der Tabelle 2 sind erfindungsgemäß. Die Mengenangaben beziehen sich auf Gew.-% der handelsüblichen Substanzen in der Gesamtzusammensetzung. | | | | |
|---|---|---|---|---|
| **Inhaltsstoffe** | **3** | **4** | **5** | **6** |
| Eumulgin® B1 | 3,5 | 3,5 | 3,5 | 3,5 |
| Cutina® PES | 2,5 | 2,5 | 2,5 | 2,5 |
| Cetiol® OE | | | | |
| Cetiol® LC | 10 | | | |
| Cetiol® 868 | | 10 | | |
| Cetiol® CC | | | 10 | |
| Cetiol® SN | | | | 10 |
| Glycerine | 3,0 | 3,0 | 3,0 | 3,0 |
| Wasser, Konservierungsmittel | ad 100 | ad 100 | ad 100 | ad 100 |
| | | | | |
| Phasenstabilität bei -5°C/RT/40°C | | | | |
| Nach 1 Woche | 1/1/1 | 1/1/1 | 1/1/1 | 1/1/1 |
| Nach 2 Wochen | 1/1/1 | 1/1/1 | 1/1/1 | 1/1/1 |
| Nach 4 Wochen | 1/1/1 | 1/1/1 | 1/1/1 | 1/1/1 |
| Nach 8 Wochen | 1/1/1 | 1/1/1 | 1/1/1 | 1/1/1 |
| Nach 12 Wochen | 1/1/1 | 1/1/1 | 1/1/1 | 1/1/1 |
| | | | | |
| Makroskopische Erscheinung bei RT | | | | |
| 1 Tag nach Herstellung | 1 | 1 | 1 | 1 |
| | | | | |
| Mikroskopische Erscheinung bei RT | | | | |
| 1 Tag nach Herstellung | 1 | 1 | 1 | 1 |

| | | | | |
|---|---|---|---|---|
| RT = Raumtemperatur 20°C Legende: Bewertungskriterien für die visuelle Phasenstabilität: 1 - stabil; 2 - geringfügige Trennung; 3 - leichte Trennung; 4 - deutliche Trennung; 5 - Trennung Bewertungskriterien für die makroskopische Erscheinung: 1 - bläulich; 2 - blau-weiss; 3 - weiss; 4 -Kristallbildung | | | | |

Die Bewertung der Muster wird nach Temperieren auf Raumtemperatur durchgeführt.

Bewertungskriterien für die mikroskopische Erscheinung:
1 - durchschnittliche Partikelgröße ≤ 1µm
2 - durchschnittliche Partikelgröße 1 - 4µm
3 - durchschnittliche Partikelgröße 4 - 13µm
4 - durchschnittliche Partikelgröße 13 - 20µm
5 - durchschnittliche Partikelgröße 20 - 50µm

Die Partikelgröße der Testemulsion wurde visuell mit der Partikelgröße von Standardemulsionen verglichen. Zur Bestimmung der Partikelgröße der Standardemulsionen wird mittels Laserbeugung ein -Beugungsmuster ermittelt. Aus den Lichtintensitäten dieser Beugungsmuster wird dann mittels der Fraunhofer-Theorie die Teilchengrößenverteilung errechnet (Sympatec Helos).

**Tabelle 3**

| Die Rezepturen 7 -14 in der Tabelle 3 sind erfindungsgemäß. Die Mengenangaben beziehen sich auf Gew.-% der handelsüblichen Substanzen in der Gesamtzusammensetzung. | | | | | | | | |
|---|---|---|---|---|---|---|---|---|
| **Inhaltsstoffe** | **7** | **8** | **9** | **10** | **11** | **12** | **13** | **14** |
| Eumulgin® B1 | 3,5 | 2,9 | 3,5 | 3,5 | 3,5 | 3,5 | 3,5 | 3,5 |
| Eumulgin® B2 | | 1,0 | | | | | | |
| Cutina® PES | 2,5 | 2,1 | 2,5 | 2,5 | 2,5 | 2,5 | 2,5 | 2,5 |
| Cetiol® OE | | | | | | | | |
| Cetiol® LC | | | 5,0 | 7,5 | 7,5 | 7,5 | 7,5 | 7,5 |
| Cetiol® PGL | 10 | 10 | | | | | | |
| Eutanol® G16 | | | 5,0 | | | | | |
| Myritol® 331 | | | | 2,5 | | | | |
| Cetiol® B | | | | | 2,5 | | | |
| Cetiol® AB | | | | | | 2,5 | | |
| Ethylhexylsalicylate | | | | | | | 2,5 | |
| Octocrylene | | | | | | | | 2,5 |
| Glycerine | 3,0 | 3,0 | 3,0 | 3,0 | 3,0 | 3,0 | 3,0 | 3,0 |
| Wasser, | ad | ad | ad | ad | ad | ad | ad | ad |
| Konservierungsmittel | 100 | 100 | 100 | 100 | 100 | 100 | 100 | 100 |
| | | | | | | | | |
| Phasenstabilität bei -5°C/RT/40°C | | | | | | | | |
| Nach 1 Woche | 1/1/1 | 1/1/1 | 1/1/1 | 1/1/1 | 1/1/1 | 1/1/1 | 1/1/1 | 1/1/1 |
| Nach 2 Wochen | 1/1/1 | 1/1/1 | 1/1/1 | 1/1/1 | 111/1 | 1/1/1 | 1/1/1 | 1/1/1 |
| Nach 4 Wochen | 1/1/2 | 1/1/2 | 1/1/2 | 1/1/1 | 1/1/1 | 1/1/1 | 1/1/1 | 1/1/1 |
| Nach 8 Wochen | 1/1/3 | 1/1/3 | 1/1/3 | 1/1/1 | 1/1/1 | 1/1/1 | 1/1/1 | 1/1/1 |
| Nach 12 Wochen | 1/1/5 | 1/1/5 | 1/1/5 | 1/1/1 | 1/1/1 | 1/1/1 | 1/1/1 | 1/1/1 |
| | | | | | | | | |
| Makr. Erscheinung bei RT | | | | | | | | |
| 1 Tag nach Herstellung | 1 | 1 | 1 | 1 | 1 | 1 | 1 | 1 |
| Mikr. Erscheinung bei RT | | | | | | | | |
| 1 Tag nach Herstellung | 1-2 | 1-2 | 1 | 1 | 1 | 1 | 1 | 1 |

| | | | | | | | | |
|---|---|---|---|---|---|---|---|---|
| Makr. Erscheinung = Makroskopische Erscheinung Mikr. Erscheinung = Mikroskopische Erscheinung RT = Raumtemperatur 20°C Legende: Bewertungskriterien für die visuelle Phasenstabilität: 1 - stabil; 2 - geringfügige Trennung; 3 - leichte Trennung; 4 - deutliche Trennung; 5 - Trennung Bewertungskriterien für die makroskopische Erscheinung: 1 - bläulich; 2 - blau-weiss; 3 - weiss; 4 -Kristallbildung | | | | | | | | |

Die Bewertung der Muster wird nach temperieren auf Raumtemperatur durchgeführt.

Bewertungskriterien für die mikroskopische Erscheinung:
1- durchschnittliche Partikelgröße ≤ 1µm
2 - durchschnittliche Partikelgröße 1 - 4µm
3 - durchschnittliche Partikelgröße 4 - 13µm
4 - durchschnittliche Partikelgröße 13 - 20µm
5 - durchschnittliche Partikelgröße 20 - 50µm

Die Partikelgröße der Testemulsion wurde visuell mit der Partikelgröße von Standardemulsionen verglichen. Zur Bestimmung der Partikelgröße der Standardemulsionen wird mittels Laserbeugung ein Beugungsmuster ermittelt. Aus den Lichtintensitäten dieser Beugungsmuster wird dann mittels der Fraunhofer-Theorie die Teilchengrößenverteilung errechnet (Sympatec Helos).

**Tabelle 4**

| Die Rezeptur 15 in der Tabelle 4 ist erfindungsgemäß, die Rezeptur V4 dient zum Vergleich. Die Mengenangaben beziehen sich auf Gew.-% der handelsüblichen Substanzen in der Gesamtzusammensetzung. | | |
|---|---|---|
| **Inhaltsstoffe** | **V4** | **15** |
| Emulgade® SE-PF | 7,8 | |
| Eumulgin® B1 | | 4,5 |
| Eumulgin® B3 | 5,2 | 5,2 |
| Cutina® PES | | 3,3 |
| Cetiol® OE | 2,0 | 2,0 |
| Cetiol® SN | 2,0 | 2,0 |
| Benzophenone-3 | 4,0 | 4,0 |
| Homosalat | 7,0 | 7,0 |
| Ethylhexyl Methoxycinnamat | 7,5 | 7,5 |
| Ethylhexyl Salicylat | 5,0 | 5,0 |
| Copherol® F 1300 | 1,0 | 1,0 |
| Glycerin | 5,0 | 5,0 |
| Wasser, Konservierungsmittel | ad 100 | ad 100 |
| | | |
| Phasenstabilität bei -5°C/RT/40°C | | |
| Nach 1 Woche | 1/1/1 | 1/1/1 |
| Nach 2 Wochen | 1/1/1 | 1/1/1 |
| Nach 4 Wochen | 1/1/1 | 1/1/1 |
| Sensorische Bewertung der RT Muster | 2 | 1 |

| | | |
|---|---|---|
| RT = Raumtemperatur 20°C Legende: Bewertungskriterien für die visuelle Phasenstabilität: 1 - stabil; 2 - geringfügige Trennung; 3 - leichte Trennung; 4 - deutliche Trennung 5 - Trennung Sensorische Bewertungskriterien (10 Probanden) 1- sehr hohe Akzeptanz; 2 - durchschnittliche Akzeptanz; 3 - nicht akzeptabel Beispiel 16 Emulgator Compound 60 Gew.-% Eumulgin B1 40 Gew.-% Cutina PES | | |

### Anhang

| | | | |
|---|---|---|---|
| 1) | Cetiol® AB | | Hersteller: Cognis Deutschland GmbH & |
| | INCI: C12-15 Alkylbenzoate | | Co. KG |
| | Hersteller: Cognis Deutschland GmbH & | | |
| | Co. KG | 8) | Cetiol® PGL |
| | | | INCI: Hexyldecanol + Hexyldecyl Laurate |
| 2) | Cetiol® B | | Hersteller: Cognis Deutschland GmbH & |
| | INCI: Dibutyl Adipate | | Co. KG |
| | Hersteller: Cognis Deutschland GmbH & | | |
| | Co. KG | 9) | Copherol® F 1300 |
| | | | INCI: Tocopherol |
| 3) | Cetiol® CC | | Hersteller: Cognis Deutschland GmbH & |
| | INCI: Dicaprylyl Carbonate | | Co. KG |
| | Hersteller: Cognis Deutschland GmbH & | | |
| | Co. KG | | 10) Cutina® MD |
| | | | INCI: Glyceryl Stearat |
| 4) | Cetiol® LC | | Hersteller: Cognis Deutschland GmbH & |
| | INCI: Coco-Caprylate/Caprat | | Co. KG |
| | Hersteller: Cognis Deutschland GmbH & | | |
| | Co. KG | 11) | Cutina® GMS-V |
| | | | INCI: Glyceryl Stearat |
| 5) | Cetiol® OE | | Hersteller: Cognis Deutschland GmbH & |
| | INCI: Dicaprylyl Ether | | Co. KG |
| | Hersteller: Cognis Deutschland GmbH & | | |
| | Co. KG | 12) | Cutina® PES |
| | | | INCI: Pentaerythrityl Distearat |
| 6) | Cetiol® SN | | Hersteller: Cognis Deutschland GmbH & |
| | INCI: Cetearyl Isononanoate | | Co. KG |
| | Hersteller: Cognis Deutschland GmbH & | | |
| | Co. KG | 13) | Emulgade® SE-PF |
| | | | INCI: Glyceryl Stearat, Ceteareth-20, |
| 7) | Cetiol® 868 | | Ceteareth-12, Cetearyl Alcohol, Cetyl |
| | INCI: Ethylhexyl Stearate | | Palmitat |
| | Hersteller: Cognis Deutschland GmbH & | | |
| | Co. KG | | |
| 14) | Eumulgin® B1 | | |
| | INCI: Ceteareth-12 | | |
| | Hersteller: Cognis Deutschland GmbH & | | |
| | Co. KG | | |
| 15) | Eumulgin® B2 | | |
| | INCI: Ceteareth-20 | | |
| | Hersteller: Cognis Deutschland GmbH & | | |
| | Co. KG | | |
| 16) | Eumulgin® B3 | | |
| | INCI: Ceteareth-30 | | |
| | Hersteller: Cognis Deutschland GmbH & | | |
| | Co. KG | | |
| 17) | Eutanol® G16 | | |
| | INCI: Hexyldecanol | | |
| | Hersteller: Cognis Deutschland GmbH & | | |
| | Co. KG | | |
| 18) | Myritol® 331 | | |
| | INCI: Cocoglycerides | | |
| | Hersteller: Cognis Deutschland GmbH & | | |
| | Co. KG | | |

## Patentansprüche

1. Emulgatorzusammensetzung enthaltend
(a) wenigstens einen Ester des Pentaerythrits, des Dipentaerythrits, des Tripentaerythrits oder eines bellebigen Gemisches dieser Ester, wobei die Ester ein Gemisch verschledener Fettsäure-Acylgruppen aufweisen **dadurch gekennzeichnet, dass** die Komponente (a) ausgewählt ist aus der Gruppe der C8-22- Fettsäureester des Pentaerythrit, des Dipentaerythrits, des Tripentaerythrits oder eines beliebigen Gemisches dieser Ester, die einen Schmelzpunkt von wenigstens 30°C aufweisen.
(b) wenigstens einen alkoxylierten nichtionischen Emulgator und
(c) weniger als 10 Gew.-% Wasser,
**dadurch gekennzeichnet, dass** falls die Zusammensetzung Partialglyceride enthält, diese in einer Menge kleiner gleich 10 Gew.-% bezogen auf die Menge an Pentaerythritester (e) vorliegen.

2. Emulgatorsammensetzung gemäß Anspruch 1, **dadurch gekennzeichnet, dass** das Gewichtsverhälltnis von (a);(b) zwischen 20 : 80 - 80 : 20, vorzugsweise 25 : 75 - 75 : 25, bevorzugt 30 : 70 - 70 : 30, vorzugsweise 35 : 65 - 65 : 36 variiert.

3. Emulgatorzusammensetzung gemäß wenigstens einem der Ansprüche 1 bis 2, **dadurch gekennzeichnet, dass** die Komponente (a) ein Pentaerythritestergemisch mit einen Anteil von (i) 5-35 Gew.-% Monoester, (ii) 20 - 50 Gew.-% Diester und (iii) 25 - 50 Gew.-% Triester, und ggf. Tetraester ist.

4. Emulgatorzussmmensetzung gemäß wenigstens einem der Ansprüche 1 bis 3 **dadurch gekennzeichnet, dass** die Komponente (a) ein Pentaerythritestergemisch mit einen Anteil von (1) 10 -25 Gew.-% Monoester, (ii) 25 - 40 Gew.-% Diester, (iii) 30 - 45 Gew.-% Triester und ggf. Tetraester ist, vorzugeweise mit einem Anteil von (i) 12 -19 Gew.-% Monoester, (ii) 25 - 36 Gew.-% Diester. (iii) 30 - 40 Gew.-% Triester und (iv) 8-11 Gew.-% Tetraester.

5. Emulgatorzusammensetzung gemäß wenigstens einem der Ansprüche 1 bis 4 **dadurch gekennzeichnet, dass** die Komponente (b) ausgewählt ist aus den ethoxylierten nichtionischen Emulgaloren, vorzugswelse aus der Gruppe der C12-C24-Fettalkoholethoxylate oder einem bellebigen Gemisch von C12-C24-Fettalkoholethoxylalen.

6. Verwendung einer Emulgatorzusammensetzung gemäß wenigstens einem der Ansprüche 1 bis 5 In Emulsionen.

7. Verwendung einer Emulgatorzusammensetzung gemäß wenigstens einem der Ansprüche 1 bis 5. als selbslemulgierende Grundlage zur Herstellung von Emulsionen.

8. Verwendung einer Emulgatorzusammensetzung gemäß wenigstens einem der Ansprüche 1 bis 5 zur Herstellung von PIT- und Mikroemulsionen, vorzugsweise mit einer mittleren Tröpfchengrößenvertellung von kleiner gleich 100Q nm, bevorzugt kleiner gleich 600 nm, ganz besonders bevorzugt kleiner gleich 260 nm, bevorzugt kleiner gleich 200 nm.

9. Verfahren zur Herstellung von Emulsionen, wobei man
(a) eine Emulgatorzusammensetzung gemäß einem der Ansprüche 1 bis 5 in einer Ölphase ggf. enthaltend weitere öllösliche Komponenten dispergiert und diese auf eine Temperatur oberhalb der Phaseninverslonstemperatur erwärmt,
(b) eine wässrige Phase enthaltend ggf. wasserlöslich Bestandteile ebenfalls auf eine Temperatur oberhalb der Phaseninveraionstemperatur erwärmt, und dann
(c) die Ölphase und die wässrige Phase zusammen unter Rühren auf Raumtemperatur abkühlt.

10. Kosmetische. Zusammensetzung enthaltend 0,6 - 40, bevorzugt 2 - 20 Gew.-% einer Emulgatorzusammensetzung gemäß wenigstens einem der Ansprüche 1 bis 5.

11. Zusammensetzung gemäß Anspruch 10, **dadurch gekennzeichnet, dass** zusätzlich wenigstens eine bei 20 °C flüssige Ölkomponente enthalten ist.

12. Kosmetische Zusammensetzung gemäß wenigstens einem der Ansprüche 10 bis 11, **dadurch gekennzeichnet, dass**
(a) 0,5 - 40, bevorzugt 2 - 20 Gew,-% einer Emulgatorzusammansetzung gemäß wenigstens einem der Ansprüche 1 bis 5
(b) 5-30 Gew.-% wenigstens einer bei 20 °C flüssigen Ölkomponente und
(c) Wasser
enthalten ist.

13. Verwandung einer Zusammensetzung gemäß einem der Ansprüche 10 bis 12 zur Beschichtung von Vilesen, Geweben, Kosmetik- und Haushaltstüchem.

14. Verwendung nach Anspruch 13 als Avivagemittel.

15. Substrate, die mit einer kosmetischen Zusammensetzung gemäß einem der Ansprüche 10.bis 12 beschichtet sind.

## Claims

1. Emulsifier composition containing
(a) at least one ester of pentaerythritol, dipentaerythritol, tripentaerythritol or a mixture of these esters, wherein the ester comprise a mixture of different fatty acid acyl groups, **characterized in that** component (a) is selected from the group of C6-C22 fatty acid esters of pentaerythritol, dipentaerythritol, tripentaerythritol or a mixture of these esters which have a melting point of at least 30°C
(b) at least one alkoxylated nonionic emulsifier and
(c) less than 10% by weight water,
**characterized in that**, if this composition contains partial glycerides, they are present in a quantity of, or less than, 10% by weight, based on the quantity of pentaerythritol ester (a).

2. Emulsifier composition as claimed in claim 1, **characterized in that** the ratio by weight of (a) to (b) varies from 20:80 to 80:20, preferably from 25:75 to 75:25, more preferably from 30:70 to 70:30 and most preferably from 35:65 to 65:35.

3. Emulsifier composition as claimed in at least one of claims 1 to 2, **characterized in that** component (a) is a pentaerythritol ester mixture containing (i) 5 to 35% by weight monoester, (ii) 20 to 50% by weight diester and (iii) 25 to 50% by weight triester and optionally tetraester.

4. Emulsifier composition as claimed in at least one of claims 1 to 3, **characterized in that** component (a) is a pentaerythritol ester mixture containing (i) 10 to 25% by weight monoester, (ii) 25 to 40% by weight diester, (iii) 30 to 45% by weight triester and optionally tetraester, preferably (i) 12 to 19% by weight monoester, (ii) 25 to 35% by weight diester, (iii) 30 to 40% by weight triester and (iv) 6 to 11 % by weight tetraester.

5. Emulsifier composition as claimed in at least one of claims 1 to 4, **characterized in that** component (b) is selected from the ethoxylated nonionic emulsifiers, preferably from the group of C12-C24 fatty alcohol ethoxylates or a mixture of C12-C24 fatty alcohol ethoxylates.

6. Use of the emulsifier composition claimed in at least one of claims 1 to 5 in emulsions.

7. Use of the emulsifier composition claimed in at least one of claims 1 to 5 as a self-emulsifying base for the production of emulsions.

8. Use of the emulsifier composition claimed in at least one of claims 1 to 5 for the production of PIT and microemulsions, preferably with a mean droplet size distribution of or smaller than 1000 nm, preferably of or smaller than 500 nm, more preferably of or smaller than 250 nm and most preferably of or smaller than 200 nm.

9. Process for the production of emulsions in which
(a) an emulsifier composition according to any of claims 1 to 5 is dispersed in an oil phase optionally containing other oil-soluble components and the dispersion is heated to a temperature above the phase inversion temperature,
(b) an aqueous phase optionally containing water-soluble constituents is also heated to a temperature above the phase inversion temperature and
(c) the oil phase and the aqueous phase are then cooled together with stirring to room temperature.

10. Cosmetic composition containing 0.5 to 40, preferably 2 to 20% by weight of the emulsifier composition claimed in at least one of claims 1 to 5.

11. Cosmetic composition as claimed in claim 10, **characterized in that** at least one oil component liquid at 20°C is additionally present.

12. Cosmetic composition as claimed in at least one of claims 10 to 11, **characterized in that** it contains
(a) 0.5 to 40% by weight and preferably 2.0 to 20% by weight of the emulsifier composition claimed in at least one of claims 1 to 5,
(b) 5 to 30% by weight of at least one oil component liquid at 20°C and
(c) water.

13. Use of the composition claimed in any of claims 10 to 12 for coating nonwovens, woven fabrics, cosmetic and household wipes.

14. Use claimed in claim 13 as a conditioner.

15. Substrates coated with the cosmetic composition claimed in any of claims 10 to 12.

## Revendications

1. Composition d'émulsifiant, contenant
(a) au moins un ester du pentaérythritol, du dipentaérythritol, du tripentaérythritol ou un mélange quelconque de tels esters, les esters comportant un mélange de différents groupes acyle d'acides gras, **caractérisée en ce que** le composant (a) est choisi dans le groupe des esters d'acides gras en C₆-C₂₂ du pentaérythritol, du dipentaérythritol, du tripentaérythritol ou d'un mélange quelconque de ces esters, qui ont un point de fusion inférieur à 30 °C,
(b) au moins un émulsifiant non ionique alcoxylé et
(c) moins de 10 % en poids d'eau,
**caractérisée en ce que** lorsque la composition contient des glycérides partiels, ces derniers sont présents en une quantité inférieure ou égale à 10 % en poids, par rapport à la quantité d'ester de pentaérythritol (a).

2. Composition d'émulsifiant selon la revendication 1, **caractérisée en ce que** le rapport pondéral de (a):(b) varie entre 20:80 et 80:20, de préférence entre 25:75 et 75:25, encore mieux entre 30:70 et 70:30, de préférence entre 35:65 et 65:35.

3. Composition d'émulsifiant selon au moins l'une des revendications 1 et 2, **caractérisée en ce que** le composant (a) est un mélange d'esters de pentaérythritol ayant une teneur (i) en monoester de 5-35 % en poids, (ii) en diester de 20-50 % en poids et (iii) en triester de 25-50 % en poids et éventuellement une certaine quantité de tétraester.

4. Composition d'émulsifiant selon au moins l'une des revendications 1 à 3, **caractérisée en ce que** le composant (a) est un mélange d'esters de pentaérythritol ayant une teneur (i) en monoester de 10-25 % en poids, (ii) en diester de 25-40 % en poids, (iii) en triester de 30-45 % en poids et éventuellement une certaine quantité de tétraester, de préférence ayant une teneur (i) en monoester de 12-19 % en poids, (ii) en diester de 25-35 % en poids, (iii) en triester de 30-40 % en poids et (iv) en tétraester de 8-11 % en poids.

5. Composition d'émulsifiant selon au moins l'une des revendications 1 à 4, **caractérisée en ce que** le composant (b) est choisi parmi les émulsifiants non ioniques éthoxylés, de préférence dans le groupe des éthoxylates d'alcools gras en C₁₂-C₂₄ ou un mélange quelconque d'éthoxylates d'alcools gras en C₁₂-C₂₄.

6. Utilisation d'une composition d'émulsifiant selon au moins l'une des revendications 1 à 5, dans des émulsions.

7. Utilisation d'une composition d'émulsifiant selon au moins l'une des revendications 1 à 5, en tant que base auto-émulsionnante pour la préparation d'émulsions.

8. Utilisation d'une composition d'émulsifiant selon au moins l'une des revendications 1 à 5, pour la préparation d'émulsions PIT et de microémulsions, de préférence ayant une distribution moyenne de tailles de gouttelettes inférieure ou égale à 1 000 nm, de préférence inférieure ou égale à 600 nm, de façon tout particulièrement préférée, inférieure ou égale à 260 nm, de préférence inférieure ou égale à 200 nm.

9. Procédé pour la préparation d'émulsions, dans lequel
(a) on disperse une composition d'émulsifiant selon l'une quelconque des revendications 1 à 5 dans une phase huileuse contenant éventuellement d'autres composants liposolubles et on chauffe celle-ci à une température supérieure à la température d'inversion de phase,
(b) on chauffe également à une température supérieure à la température d'inversion de phase une phase aqueuse contenant éventuellement des composants hydrosolubles, et ensuite
(c) on refroidit ensemble la phase huileuse et la phase aqueuse, sous agitation, jusqu'à la température ambiante.

10. Composition cosmétique contenant 0,6 - 40, de préférence 2 - 20 % en poids d'une composition d'émulsifiant selon au moins l'une des revendications 1 à 5.

11. Composition cosmétique selon la revendication 10, **caractérisée en ce qu'**en outre elle contient au moins un composant huileux liquide à 20 °C.

12. Composition cosmétique selon au moins l'une des revendications 10 et 11, **caractérisée en ce qu'**elle contient
(a) 0,6 - 40, de préférence 2 - 20 % en poids d'une composition d'émulsifiant selon au moins l'une des revendications 1 à 5,
(b) 5 - 30 % en poids d'au moins un composant huileux liquide à 20 °C et
(c) de l'eau.

13. Utilisation d'une composition selon l'une quelconque des revendications 10 à 12, pour l'enduction de non-tissés, de tissus, de lingettes cosmétiques et de chiffons à usage domestique.

14. Utilisation selon la revendication 13, en tant qu'agent d'avivage.

15. Substrats, qui ont été enduits avec une composition cosmétique selon l'une quelconque des revendications 10 à 12.
